(19) Europäisches Patentamt
European Patent Office
Office européen des brevets

(11) EP 1 229 791 B1

(12) **EUROPÄISCHE PATENTSCHRIFT**

(45) Veröffentlichungstag und Bekanntmachung des Hinweises auf die Patenterteilung:
**21.01.2004 Patentblatt 2004/04**

(21) Anmeldenummer: **00974473.1**

(22) Anmeldetag: **27.10.2000**

(51) Int Cl.$^7$: **A01N 43/08**

(86) Internationale Anmeldenummer:
**PCT/EP2000/010620**

(87) Internationale Veröffentlichungsnummer:
**WO 2001/033966 (17.05.2001 Gazette 2001/20)**

(54) **WIRKSTOFFKOMBINATIONEN MIT INSEKTIZIDEN UND AKARIZIDEN EIGENSCHAFTEN**

ACTIVE INGREDIENT COMBINATION HAVING INSECTICIDAL AND ACARICIDAL CHARACTERISTICS

COMBINAISON DE SUBSTANCES ACTIVES A PROPRIETES INSECTICIDES ET ACARICIDES

(84) Benannte Vertragsstaaten:
**BE DE ES FR GR IT**

(30) Priorität: **09.11.1999 DE 19953775**

(43) Veröffentlichungstag der Anmeldung:
**14.08.2002 Patentblatt 2002/33**

(73) Patentinhaber: **Bayer CropScience AG**
**40789 Monheim (DE)**

(72) Erfinder:
- **BRÜCK, Ernst**
  **51467 Bergisch Gladbach (DE)**
- **ERDELEN, Christoph**
  **42799 Leichlingen (DE)**
- **FISCHER, Reiner**
  **40789 Monheim (DE)**

(56) Entgegenhaltungen:
**EP-A- 0 528 156**
**WO-A-01/24634**
**DD-A- 161 047**
**DE-A- 2 753 426**
**DE-A- 19 913 174**
**DE-A- 19 939 395**
**US-A- 4 690 947**

- **C TOMLIN (ED): "The Pesticide Manual, Tenth Edition" , PESTICIDE MANUAL,GB,FARNHAM, BCPC, VOL. ED. 10, PAGE(S) 1335-1341 XP002099499 ISBN: 0-948404-79-5 der Index der Wirkstoffklassen**

Bemerkungen:
Die Akte enthält technische Angaben, die nach dem Eingang der Anmeldung eingereicht wurden und die nicht in dieser Patentschrift enthalten sind.

EP 1 229 791 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft neue Wirkstoffkombinationen, die aus bekannten cyclischen Ketoenole einerseits und weiteren bekannten insektiziden Wirkstoffen andererseits bestehen und sehr gut zur Bekämpfung von tierischen Schädlingen wie Insekten und unerwünschten Akariden geeignet sind.

Es ist bereits bekannt, dass bestimmte cyclische Ketoenole insektizide und akarizide Eigenschaften besitzen (EP-A-528 156). Die Wirksamkeit dieser Stoffe ist gut, lässt bei niedrigen Aufwandmengen in manchen Fällen zu wünschen übrig.

Weiterhin ist schon bekannt, dass zahlreiche Heterocyclen, Organozinn-Verbindungen, Benzoylharnstoffe und Pyrethroide insektizide und akarizide Eigenschaften besitzen (vgl. WO 93-22 297, WO 93-10 083, DE-A 2 641 343, EP-A-347 488, EP-A-210 487, US-A 3 264 177 und EP-A-234 045). Auch die Wirkung dieser Stoffe ist bei niedrigen Aufwandmengen nicht immer befriedigend.

Es wurde nun gefunden, dass Verbindungen der Formel (I-b-1)

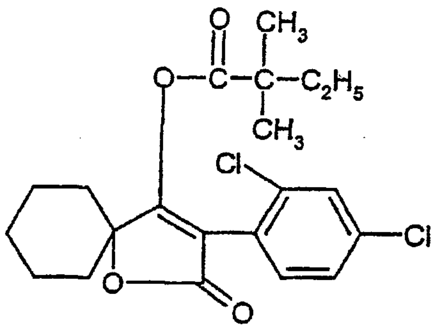

(I-b-1)

und

A) (Thio)Phosphaten, bevorzugt

1. Azinphos-methyl

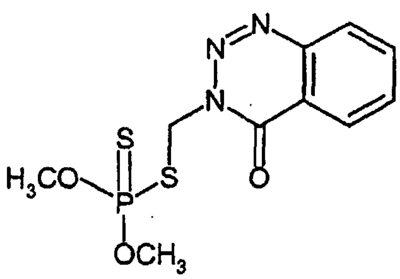

bekannt aus US 2 758 115
und/oder

2. Chlorpyrifos

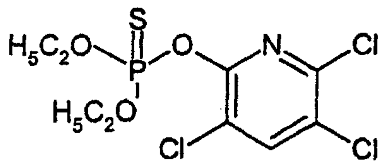

bekannt aus US 3 244 586
und/oder

3. Diazinon

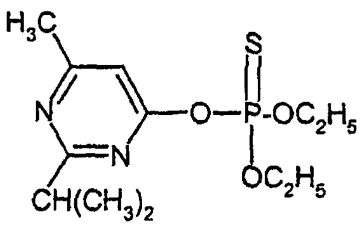

bekannt aus US 2 754 243
und/oder

4. Dimethoat

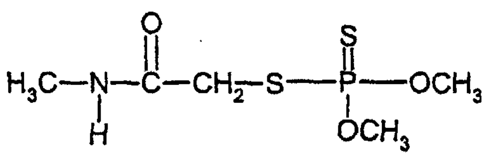

bekannt aus US 2 494 283
und/oder

5. Disulfoton

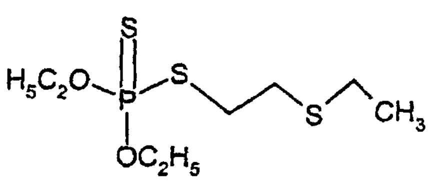

bekannt aus DE-A-917 668
und/oder

6. Ethion

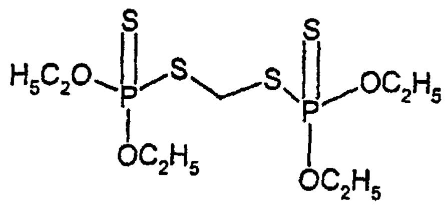

bekannt aus US 2 873 228
und/oder

7. Fenitrothion

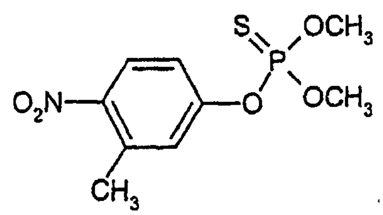

bekannt aus BE-A-594 669
und/oder

8. Fenthion

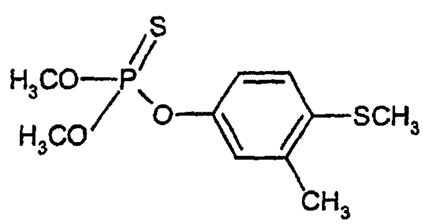

bekannt aus DE-A-1 116 656
und/oder

9. Isoxathion

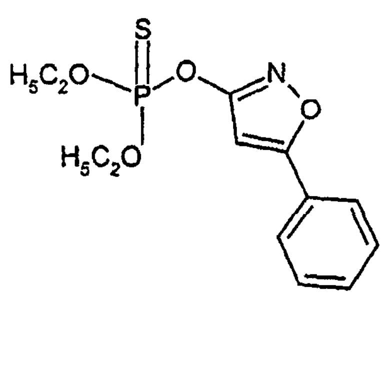

bekannt aus DE-A-1 567 137
und/oder

10. Malathion

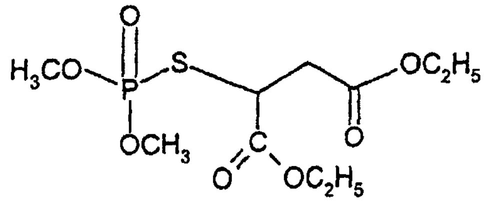

bekannt aus US 2 578 562
und/oder

11. Methidathion

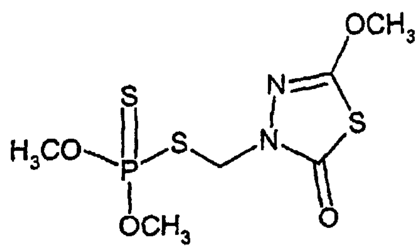

bekannt aus DE-A-1 645 982
und/oder

12. Oxydemeton-methyl

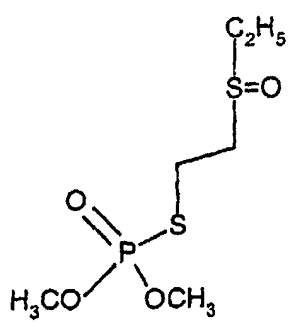

bekannt aus DE-A-947 368
und/oder

13. Parathion

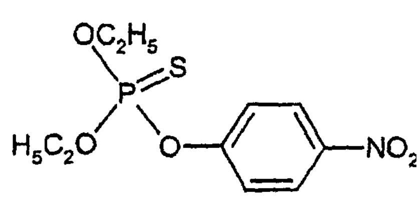

bekannt aus DE-A-814 152
und/oder

14. Parathion-methyl

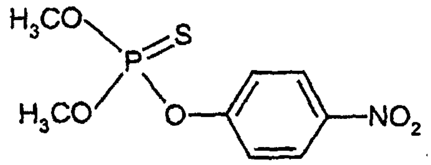

bekannt aus DE-A-814 142
und/oder

15. Phenthoat

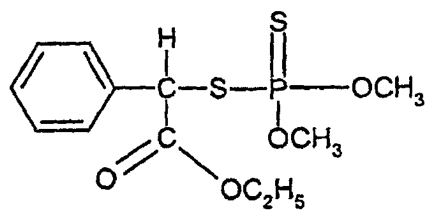

bekannt aus GB-A-834 814
und/oder

16. Phorat

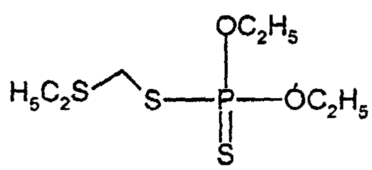

bekannt aus US 2 586 655
und/oder

17. Phosalon

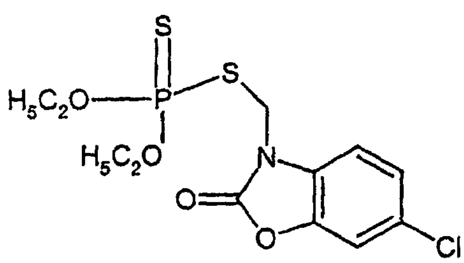

bekannt aus DE-A-2 431 192
und/oder

18. Phosmet

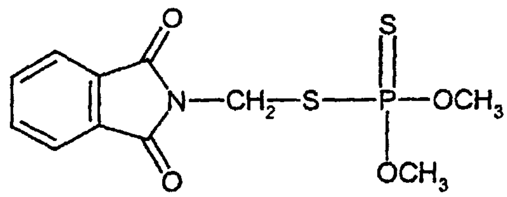

bekannt aus US 2 767 194
und/oder

19. Phoxim

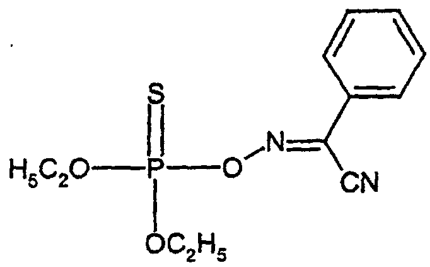

bekannt aus DE-A- 1 238 902
und/oder

20. Pirimiphos-methyl

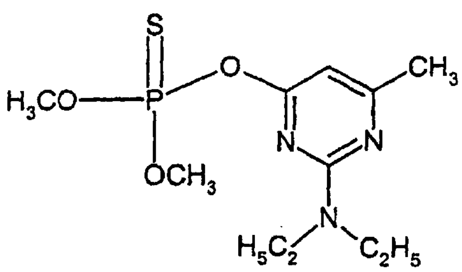

bekannt aus DE-A-1 445 949
und/oder

21. Profenophos

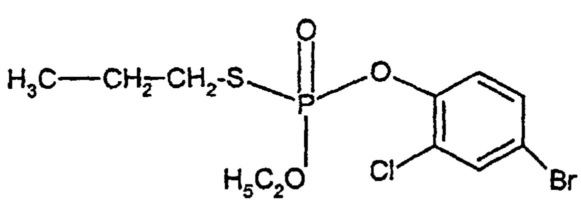

bekannt aus DE-A-2 249 462

und/oder

22. Prothiophos

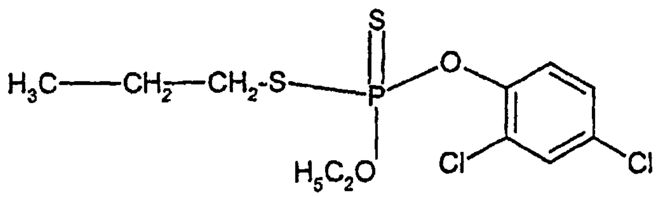

bekannt aus DE-A-2 111 414
und/oder

23. Tebupirimphos

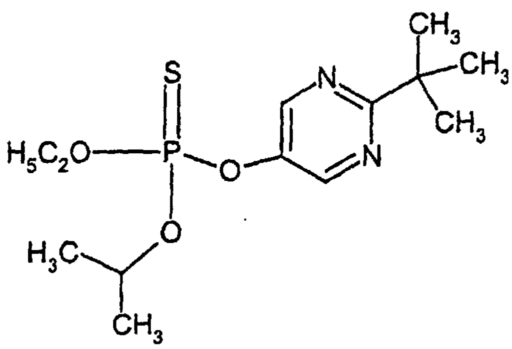

bekannt aus DE-A-3 317 824
und/oder

24. Triazophos

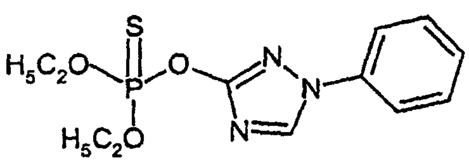

bekannt aus DE-A-1 299 924
und/oder

25. Chlorfenvinphos

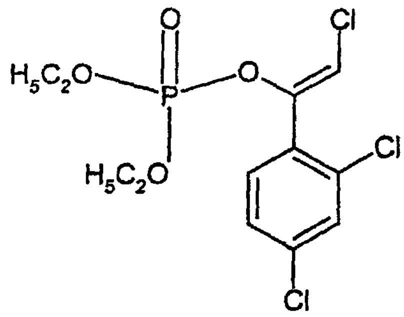

bekannt aus US-2 956 073
und/oder

26. Dichlorphos

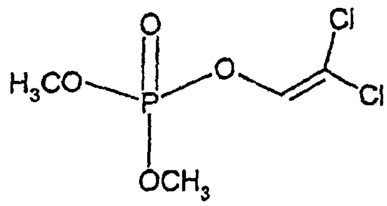

bekannt aus GB-A-775 085
und/oder

27. Dicrotophos

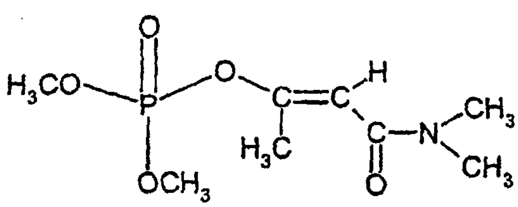

bekannt aus BE-A-55 22 84
und/oder

28. Mevinphos

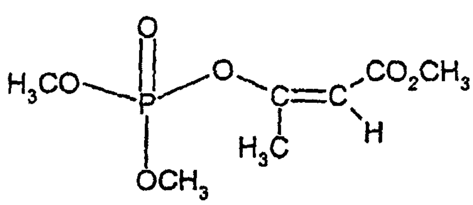

bekannt aus US-2 685 552
und/oder

29. Monocrotophos

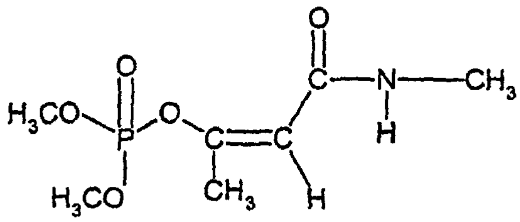

bekannt aus DE-A-1 964 535
und/oder

30. Phosphamidon

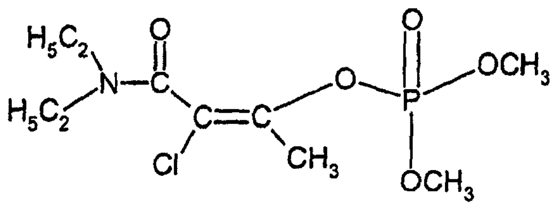

bekannt aus US 2 908 605
und/oder

31. Acephat

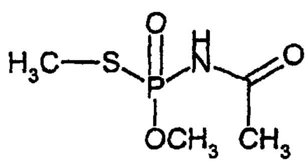

bekannt aus DE-A-2 014 027
und/oder

32. Methamidophos

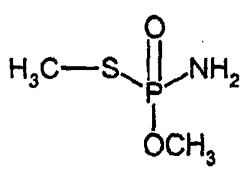

bekannt aus US-3 309 266
und/oder

33. Trichlorfon

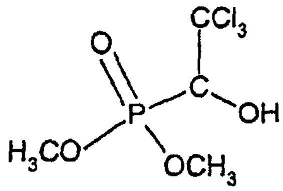

bekannt aus US-2 701 225
und/oder

B) Pyrethroiden, bevorzugt

34. Acrinathrin

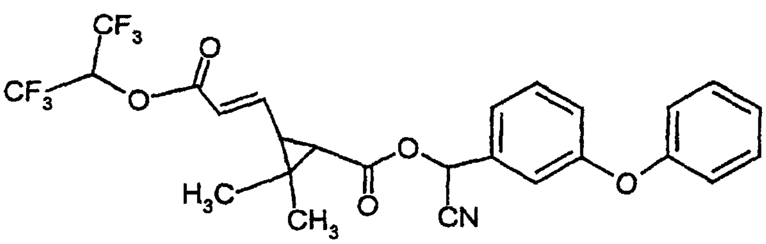

bekannt aus EP-A-048 186
und/oder

35. Alpha-cypermethrin

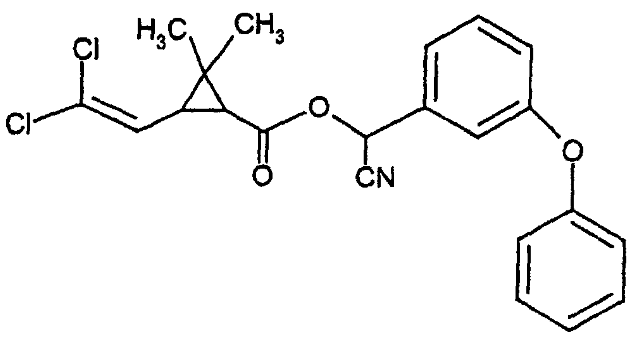

bekannt aus EP-A-067 461
und/oder

36. Betacyfluthrin

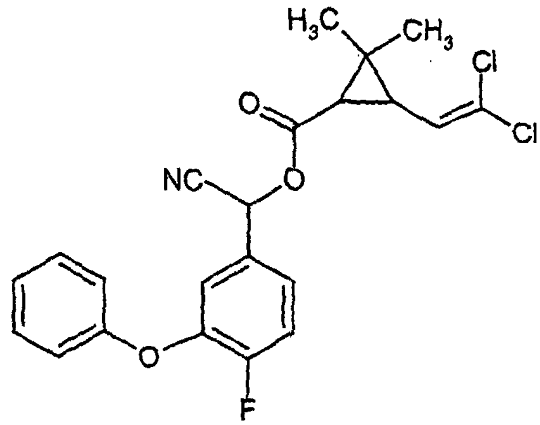

bekannt aus EP-A-206 149
und/oder

37. Cyhalothrin

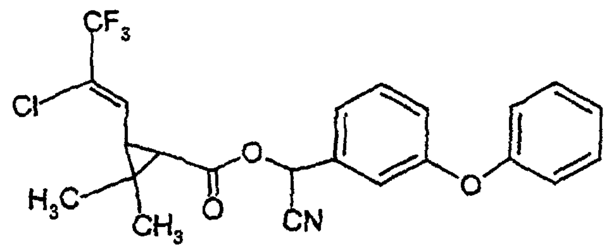

bekannt DE-A-2 802 962
und/oder

38. Cypermethrin

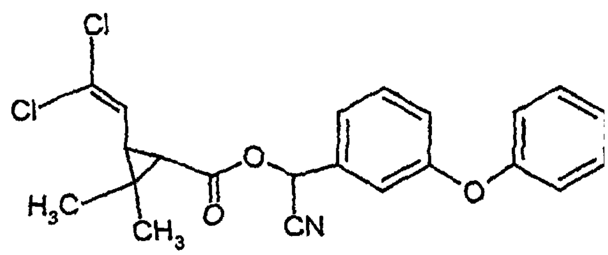

bekannt DE-A-2 326 077
und/oder

39. Deltamethrin

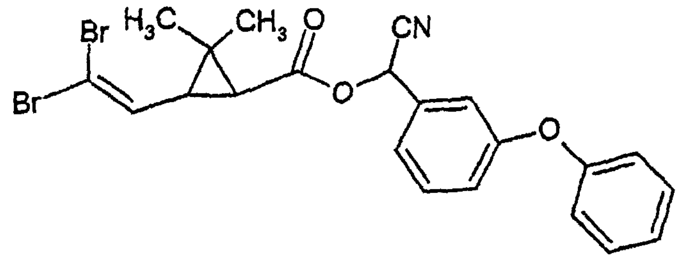

bekannt DE-A-2 326 077
und/oder

40. Esfenvalerat

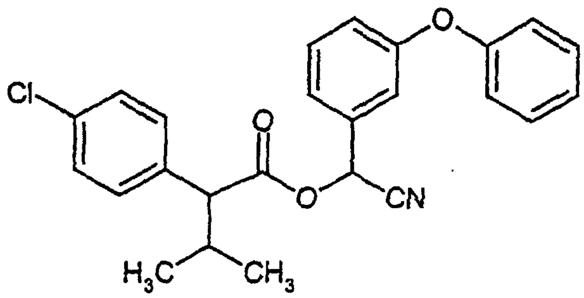

bekannt aus DE-A-2 737 297
und/oder

41. Ethofenprox

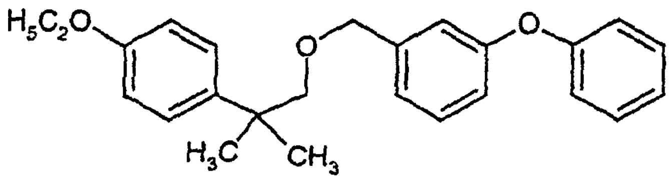

bekannt DE-A-3 117510
und/oder

42. Fenpropathrin

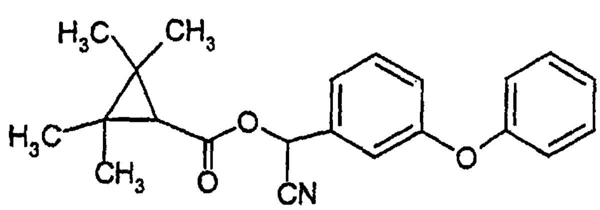

bekannt DE-A-2 231 312
und/oder

43. Fenvalerat

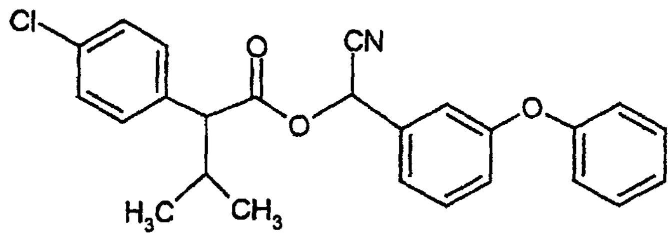

bekannt DE-A-2 335 347
und/oder

44. Flucythrinat

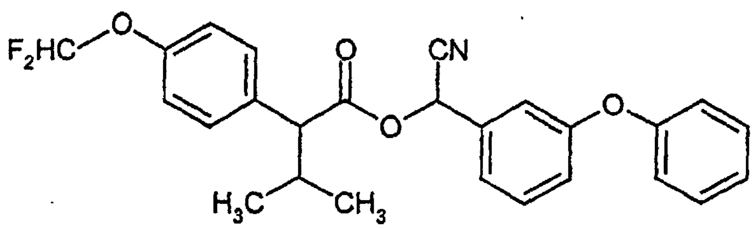

bekannt DE-A-2 757 066
und/oder

45. Lambda-cyhalothrin

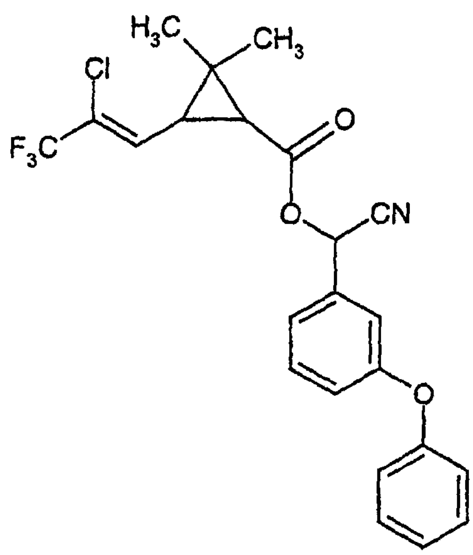

bekannt EP-A-106 469
und/oder

46. Permethrin

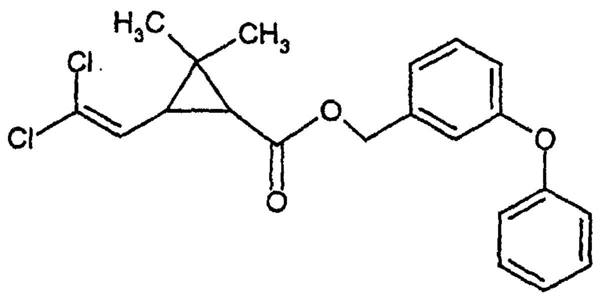

bekannt DE-A-2 326 077
und/oder

47. Taufluvalinat

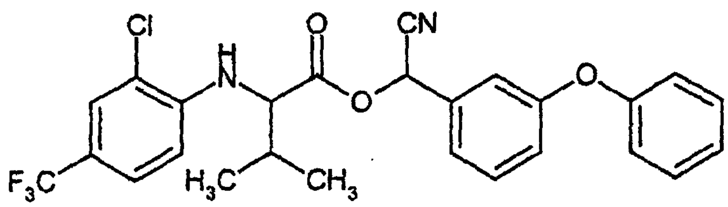

bekannt EP-A-038 617
und/oder

48. Tralomethrin

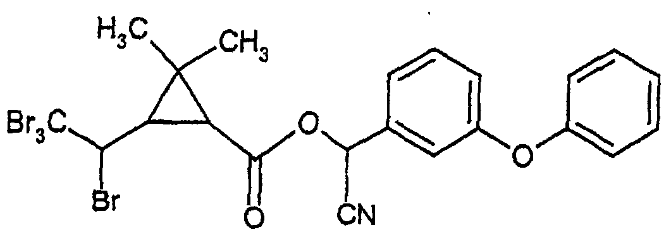

bekannt DE-A-2 742 546
und/oder

49. Zeta-cypermethrin

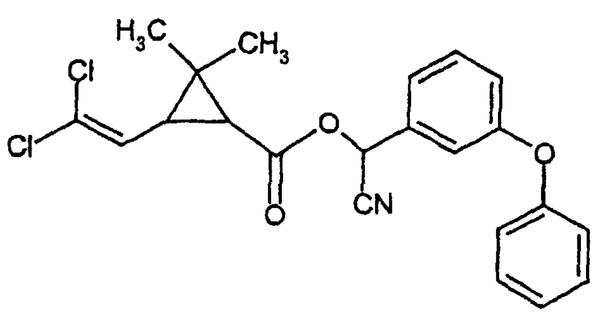

bekannt EP-A-026 542

und/oder

C) Carbamaten, bevorzugt

50. Carbaryl

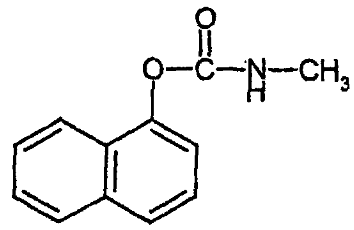

bekannt US-2 903 478
und/oder

51. Fenoxycarb

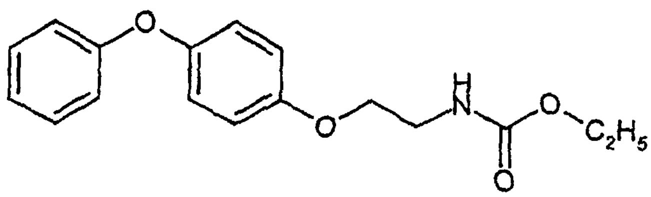

bekannt EP-A-004 334
und/oder

52. Formetanat

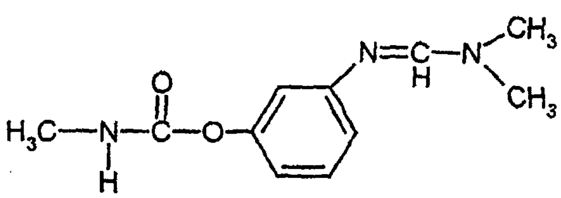

bekannt aus DE-A-1 169 194
und/oder

53. Formetanat Hydrochlorid
bekannt aus DE-A-1 169 194
und/oder

54. Methiocarb

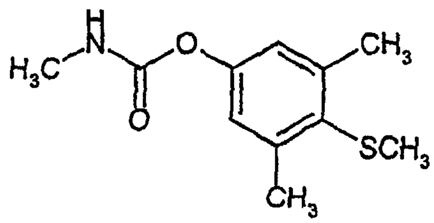

bekannt aus DE-A-1 162 352
und/oder

55. Methomyl

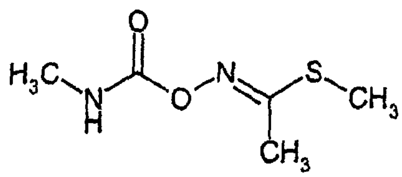

bekannt aus US- 3 639 620
und/oder

56. Oxamyl

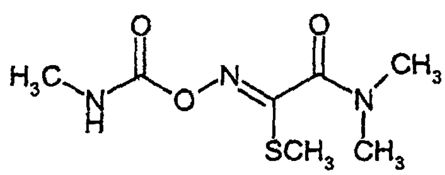

bekannt aus DE-A-1 768 623
und/oder

57. Pirimicarb

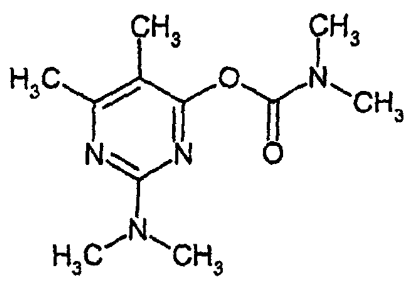

bekannt aus GB-A-1 181 657
und/oder

58. Propoxur

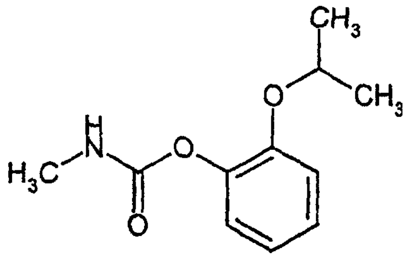

bekannt aus DE-A-1 108 202
und/oder

59. Thiodicarb

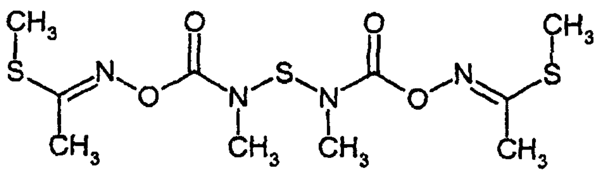

bekannt aus DE-A-2 530 439
und/oder

D.) Benzoylharnstoffen, bevorzugt

60. Chlorfluazuron

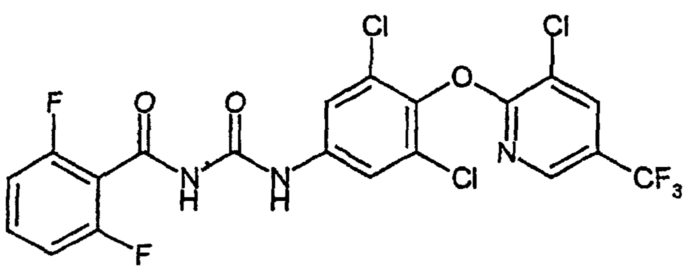

bekannt aus DE-A-2 818 830
und/oder

61. Diflubenzuron

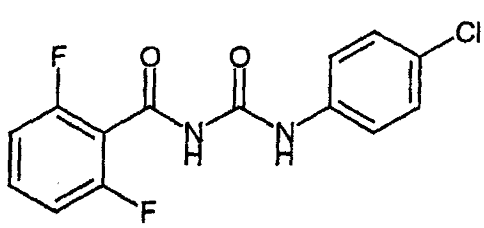

bekannt aus DE-A 2 123 236
und/oder

62. Lufenuron

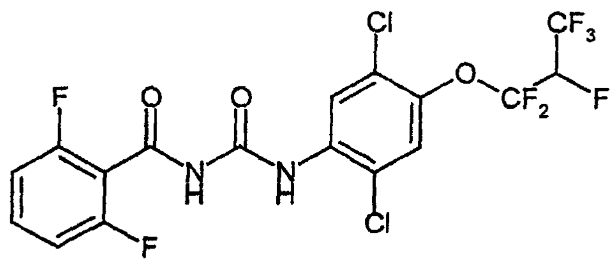

bekannt aus EP-A-179 022
und/oder

63. Teflubenzuron

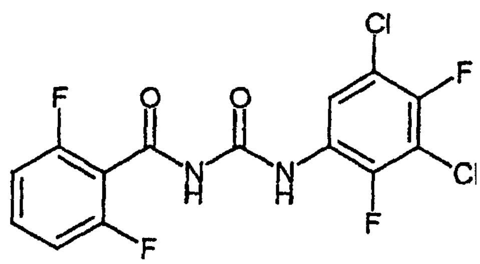

bekannt aus EP-A-052 833
und/oder

64. Triflumuron

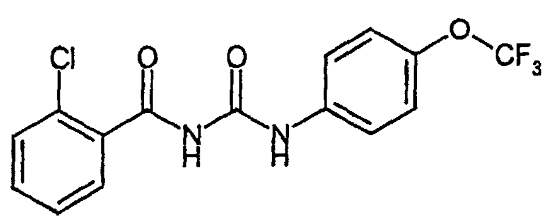

bekannt aus DE-A-2 601 780
und/oder

E) Macroliden, bevorzugt

65. Ivermectin
bekannt aus EP-A-001 689
und/oder

66. Emamectin
bekannt aus EP-A-089 202
und/oder

67. Milbemectin
bekannt aus The Pesticide Manual, 11th Edition, 1997, S. 846
und/oder

F) Diacylhydrazinen, bevorzugt

68. Methoxyfenozid

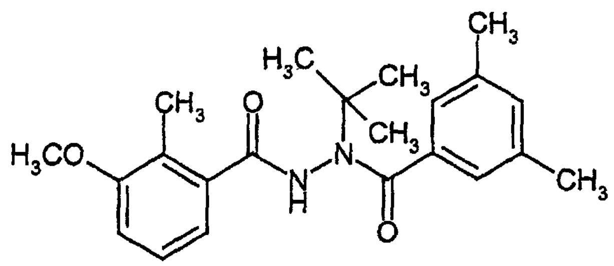

bekannt aus EP-A-639 559
und/oder

69. Tebufenozid

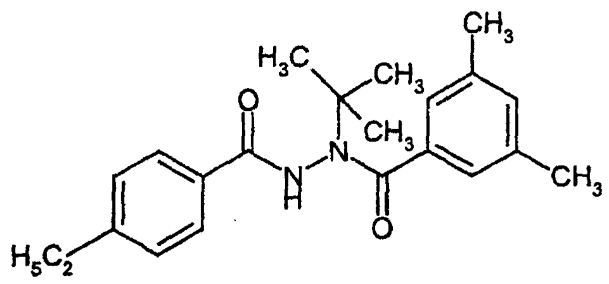

bekannt aus EP-A-339 854
und/oder

G) Halogencycloalkanen, bevorzugt

70. Endosulfan

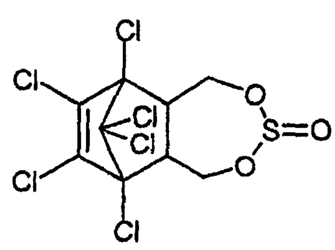

bekannt aus DE-A-1 015 797
und/oder

71. Gamma-HCH

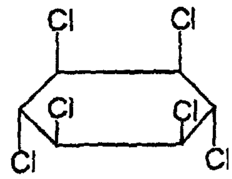

bekannt aus US 2,502,258
und/oder

H) Akariziden, bevorzugt

72. Fenazaquin

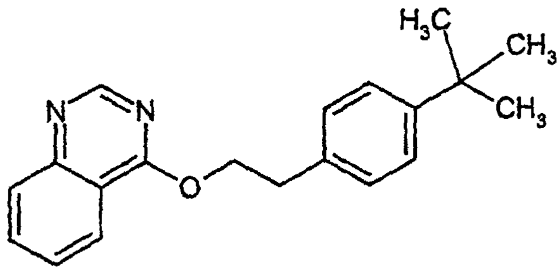

bekannt aus EP-A-326 329
und/oder

73. Tebufenpyrad

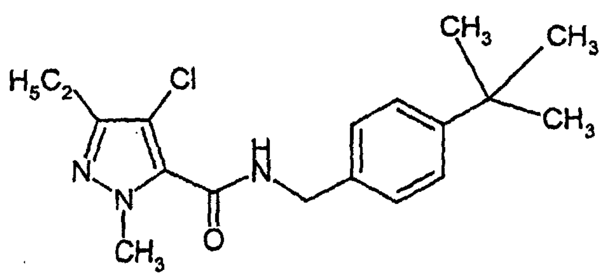

bekannt aus EP-A-289 879
und/oder

74. Pyrimidifen

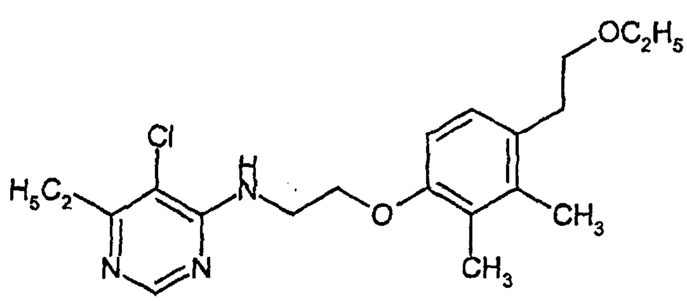

bekannt aus EP-A-196 524
und/oder

75. Triarathen

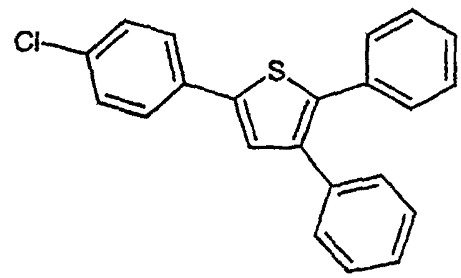

bekannt aus DE-A-2 724 494
und/oder

76. Tetradifon

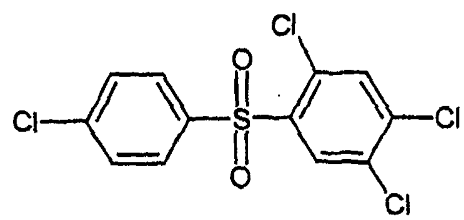

bekannt aus US 2 812 281
und/oder

77. Propargit

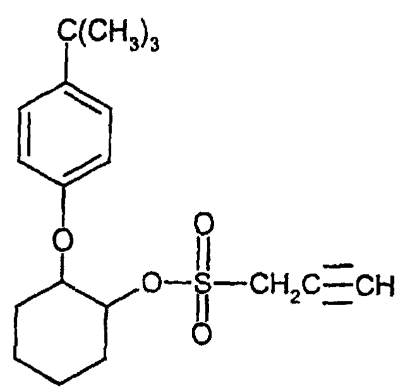

bekannt aus US 3 272 854
und/oder

78. Hexythiazox

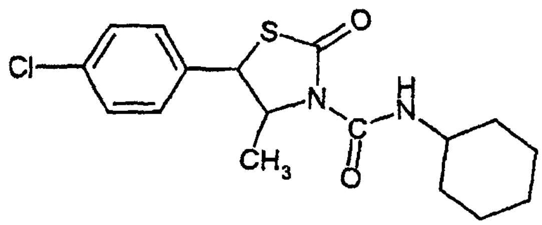

bekannt aus DE-A-3 037 105
und/oder

79. Bromopropylat

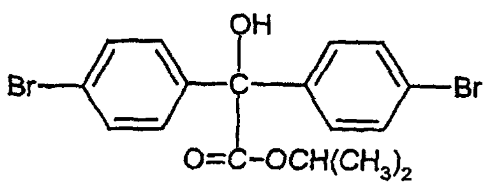

bekannt aus US 3 784 696
und/oder

80. 2-(Acetyloxy)-3-dodecyl-1,4-naphthalendion

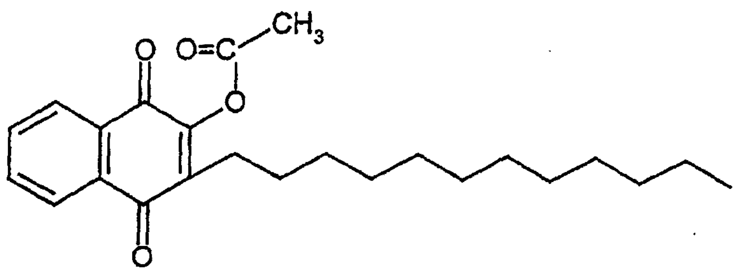

bekannt aus DE-A-2 641 343
und/oder

81. Dicofol

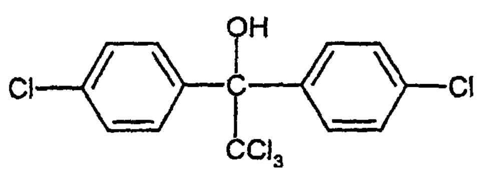

bekannt aus US 2 812 280
und/oder

l) weiteren Verbindungen wie

82. Amitraz

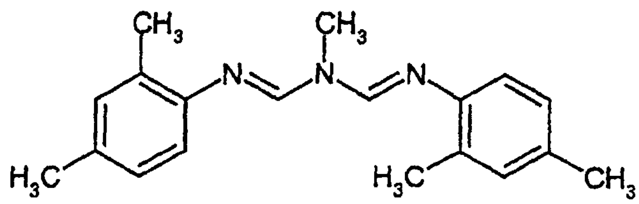

bekannt aus DE-A-2 061 132
und/oder

83. Azadirachtin
bekannt aus The Pesticide Manual, 11th Edition, 1997, S. 59

84. Buprofezin

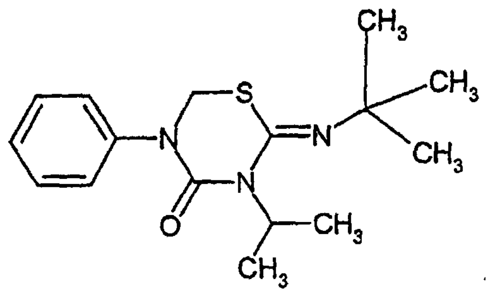

bekannt aus DE-A-2 824 126
und/oder

85. Chinomethionat

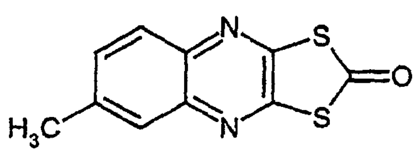

bekannt aus DE-A-1 100 372
und/oder

86. Thiocyclamhydrogenoxalat

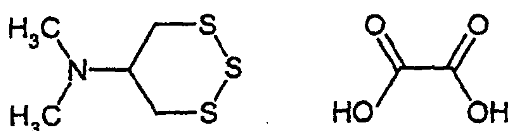

bekannt aus DE-A-2 039 666
und/oder

87. Triazamat

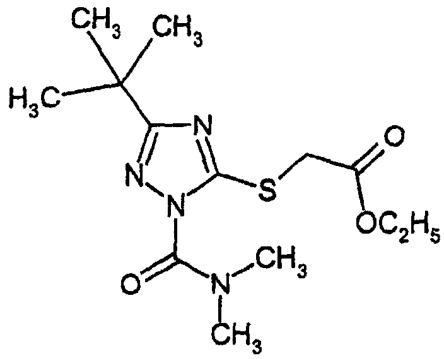

bekannt aus EP-A-213 718
und/oder

88. Trichogramma spp.
bekannt aus The Pesticide Manual, 11th Edition, 1997, S. 1236

89. Verticiliium lecanii
bekannt aus The Pesticide Manual, 11th Edition, 1997, S. 1266

90. Fipronil

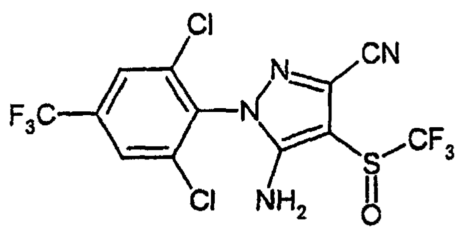

bekannt aus EP-A-295 117
und/oder

91. Cyromazin

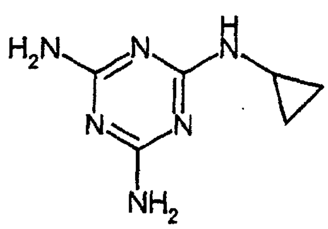

bekannt aus DE-A-2 736 876
und/oder

92. Pymetrozin

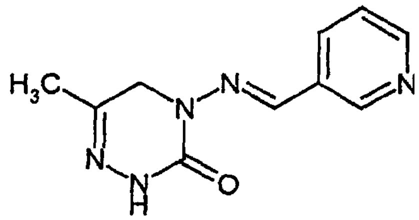

bekannt aus EP-A-314 615

93. Diofenolan

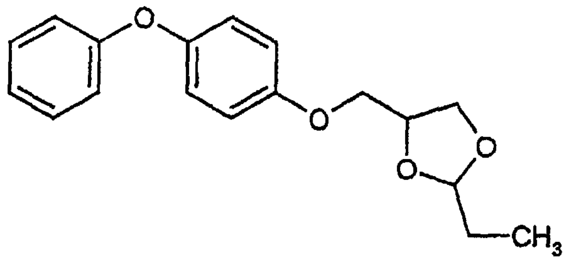

bekannt aus DE-A 2 655 910
und/oder

94. Indoxacarb

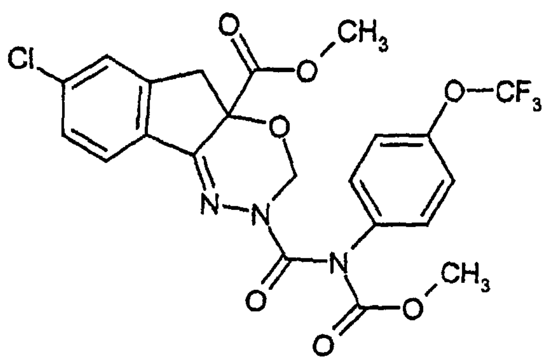

bekannt aus WO 92/11249
und/oder

95. Pyriproxyfen

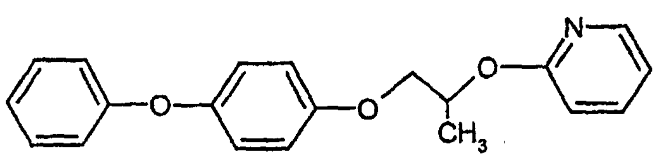

bekannt aus EP-A-128 648
sehr gute insektizide und akarizide Eigenschaften besitzen.

Überraschenderweise ist die insektizide und akarizide Wirkung der erfindungsgemäßen Wirkstoffkombination wesentlich höher als die Summe der Wirkungen der einzelnen Wirkstoffe. Es liegt ein nicht vorhersehbarer echter synergistischer Effekt vor und nicht nur eine Wirkungsergänzung.

Die erfindungsgemäßen Wirkstoffkombinationen enthalten neben mindestens einem Wirkstoff der Formel (I-b-1) mindestens einen Wirkstoff der Verbindungen 1 bis 95.

Die Wirkstoffkombinationen können darüber hinaus auch weitere fungizid, akarizid oder insektizid wirksame Zumischkomponenten enthalten.

Wenn die Wirkstoffe in den erfindungsgemäßen Wirkstoffkombinationen in bestimmten Gewichtsverhältnissen vorhanden sind, zeigt sich der synergistische Effekt besonders deutlich. Jedoch können die Gewichtsverhältnisse der Wirkstoffe in den Wirkstoffkombinationen in einem relativ großen Bereich variiert werden. Im allgemeinen enthalten die erfindungsgemäßen Kombinationen Wirkstoffe der Formel (I-b-1) und den Mischpartner in den in der nachfolgenden Tabelle angegeben bevorzugten und besonders bevorzugten Mischungsverhältnissen:

* die Mischungsverhältnisse basieren auf Gewichtsverhältnissen. Das Verhältnis ist zu verstehen als Wirkstoff der Formel (I):Mischpartner

| Mischpartner | bevorzugtes Mischungsverhältnis | besonders bevorzugtes Mischungsverhältnis |
|---|---|---|
| 2-(Acetyloxy)-3-dodecyl-1,4-naphthalinidon | 10:1 bis 1:10 | 5:1 bis 1:5 |
| Acephat | 10:1 bis 1:10 | 5:1 bis 1:5 |
| Acrinathrin | 20:1 bis 1:50 | 10:1 bis 1:1 |
| Alpha-Cypermethrin | 50:1 bis 1:5 | 10:1 bis 1:1 |
| Amitraz | 5:1 bis 1:20 | 1:1 bis 1:10 |
| Azadirachtin | 50:1 bis 1:5 | 10:1 bis 1:1 |
| Azinphosmethyl | 10:1 bis 1:10 | 5:1 bis 1:5 |
| Betacyfluthrin | 50:1 bis 1:5 | 10:1 bis 1:1 |
| Bromopropylat | 10:1 bis 1:10 | 5:1 bis 1:5 |
| Buprofezin | 10:1 bis 1:10 | 5:1 bis 1:5 |
| Carbaryl | 10:1 bis 1:10 | 5:1 bis 1:5 |
| Chinomethionat | 10:1 bis 1:10 | 5:1 bis 1:5 |
| Chlorfenvinphos | 10:1 bis 1:10 | 5:1 bis 1:5 |
| Chlorfluazuron | 10:1 bis 1:10 | 5:1 bis 1:5 |
| Chlorpyrifos | 10:1 bis 1:10 | 5:1 bis 1:5 |
| Cyhalothrin | 50:1 bis 1:5 | 10:1 bis 1:1 |
| Cypermethrin | 50:1 bis 1:5 | 10:1 bis 1:1 |
| Cyromazin | 10:1 bis 1:10 | 5:1 bis 1:5 |
| Deltamethrin | 50:1 bis 1:5 | 10:1 bis 1:1 |
| Diazinon | 10:1 bis 1:10 | 5:1 bis 1:5 |
| Dichlorphos | 10:1 bis 1:10 | 5:1 bis 1:5 |
| Dicofol | 10:1 bis 1:10 | 5:1 bis 1:5 |
| Dicrotophos | 10:1 bis 1:10 | 5:1 bis 1:5 |
| Diflubenzuron | 10:1 bis 1:10 | 5:1 bis 1:5 |
| Dimethoat | 10:1 bis 1:10 | 5:1 bis 1:5 |
| Diofenolan | 100:1 bis 1:2 | 20:1 bis 1:1 |

(fortgesetzt)

| Mischpartner | bevorzugtes Mischungsverhältnis | besonders bevorzugtes Mischungsverhältnis |
|---|---|---|
| Disulfoton | 10:1 bis 1:10 | 5:1 bis 1:5 |
| Emamectin | 50:1 bis 1:5 | 10:1 bis 1:1 |
| Endosulfan | 10:1 bis 1:10 | 5:1 bis 1:5 |
| Esfenvalerat | 50:1 bis 1:5 | 10:1 bis 1:1 |
| Ethion | 10:1 bis 1:10 | 5:1 bis 1:5 |
| Etofenprox | 10:1 bis 1:10 | 5:1 bis 1:5 |
| Fenazaquin | 10:1 bis 1:10 | 5:1 bis 1:5 |
| Fenitrothion | 10:1 bis 1:10 | 5:1 bis 1:5 |
| Fenoxycarb | 10:1 bis 1:10 | 5:1 bis 1:5 |
| Fenpropathrin | 10:1 bis 1:10 | 5:1 bis 1:5 |
| Fenpyrad (Tebufenpyrad) | 10:1 bis 1:10 | 5:1 bis 1:5 |
| Fenthion | 20:1 bis 1:10 | 5:1 bis 1:5 |
| Fenvalerat | 20:1 bis 1:5 | 10:1 bis 1:1 |
| Fipronil | 10:1 bis 1:10 | 5:1 bis 1:5 |
| Flucythrinat | 50:1 bis 1:5 | 10:1 bis 1:1 |
| Formetanat | 10:1 bis 1:10 | 5:1 bis 1:5 |
| Hexyhiazox | 20:1 bis 1:5 | 10:1 bis 1:2 |
| Indoxacarb | 50:1 bis 1:5 | 20:1 bis 1:2 |
| Isoxathion | 10:1 bis 1:10 | 5:1 bis 1:5 |
| Ivermectin | 50:1 bis 1:5 | 10:1 bis 1:1 |
| Lambda-Cyhalothrin | 50:1 bis 1:5 | 10:1 bis 1:1 |
| Lindan (Gamma-HCH) | 10:1 bis 1:10 | 5:1 bis 1:5 |
| Lufenuron | 20:1 bis 1:5 | 10:1 bis 1:2 |
| Malathion | 10:1 bis 1:10 | 5:1 bis 1:5 |
| Methamidophos | 10:1 bis 1:10 | 5:1 bis 1:5 |
| Methidathion | 10:1 bis 1:10 | 5:1 bis 1:5 |
| Methiocarb | 10:1 bis 1:10 | 5:1 bis 1:5 |
| Methomyl | 10:1 bis 1:10 | 5:1 bis 1:5 |
| Methoxyfenozid | 10:1 bis 1:10 | 5:1 bis 1:5 |
| Mevinphos | 10:1 bis 1:10 | 5:1 bis 1:5 |
| Milbemectin | 50:1 bis 1:5 | 10:1 bis 1:1 |
| Monocrotophos | 10:1 bis 1:10 | 5:1 bis 1:5 |
| Oxamyl | 5:1 bis 1:100 | 1:1 bis 1:20 |
| Oxydemeton-methyl | 10:1 bis 1:10 | 5:1 bis 1:5 |
| Parathion | 10:1 bis 1:10 | 5:1 bis 1:5 |
| Parathion-methyl | 10:1 bis 1:10 | 5:1 bis 1:5 |
| Permethrin | 10:1 bis 1:10 | 5:1 bis 1:5 |

(fortgesetzt)

| Mischpartner | bevorzugtes Mischungsverhältnis | besonders bevorzugtes Mischungsverhältnis |
|---|---|---|
| Phenthoat | 10:1 bis 1:10 | 5:1 bis 1:5 |
| Phorat | 10:1 bis 1:10 | 5:1 bis 1:5 |
| Phosalon | 10:1 bis 1:10 | 5:1 bis 1:5 |
| Phosmet | 10:1 bis 1:10 | 5:1 bis 1:5 |
| Phosphamidon | 10:1 bis 1:10 | 5:1 bis 1:5 |
| Phoxim | 10:1 bis 1:10 | 5:1 bis 1:5 |
| Pirimicarb | 10:1 bis 1:10 | 5:1 bis 1:5 |
| Pirimiphos-methyl | 10:1 bis 1:10 | 5:1 bis 1:5 |
| Profenophos | 10:1 bis 1:10 | 5:1 bis 1:5 |
| Propargit | 10:1 bis 1:10 | 5:1 bis 1:5 |
| Propoxur | 10:1 bis 1:10 | 5:1 bis 1:5 |
| Prothiophos | 10:1 bis 1:10 | 5:1 bis 1:5 |
| Pymetrozin | 10:1 bis 1:10 | 5:1 bis 1:5 |
| Pyrimidifen | 50:1 bis 1:5 | 10:1 bis 1:1 |
| Pyriproxyfen | 10:1 bis 1:10 | 5:1 bis 1:5 |
| Tau-fluvalinat | 20:1 bis 1:5 | 10:1 bis 1:2 |
| Tebufenozid | 10:1 bis 1:10 | 5:1 bis 1:5 |
| Tebupyrimphos | 10:1 bis 1:10 | 5:1 bis 1:5 |
| Teflubenzuron | 20:1 bis 1:5 | 10:1 bis 1:2 |
| Tetradifon | 10:1 bis 1:10 | 5:1 bis 1:5 |
| Thiocyclam | 5:1 bis 1:20 | 1:1 bis 1:10 |
| Thiodicarb | 5:1 bis 1:20 | 1:1 bis 1:10 |
| Tralomethrin | 50:1 bis 1:5 | 10:1 bis 1:1 |
| Triarathen | 5:1 bis 1:20 | 1:1 bis 1:10 |
| Triazamat | 10:1 bis 1:10 | 5:1 bis 1:5 |
| Triazophos | 5:1 bis 1:20 | 1:1 bis 1:10 |
| Trichlorfon | 10:1 bis 1:10 | 5:1 bis 1:5 |
| Trichogramma spp. | | |
| Triflumuron | 10:1 bis 1:10 | 5:1 bis 1:5 |
| Verticillium- lecanii | | |
| Zeta-Cypermethrin | 50:1 bis 1:5 | 10:1 bis 1:2 |

Die erfindungsgemäßen Wirkstoffkombinationen eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spp..

Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Acheta domesticus, Gryllotalpa spp., Locusta migratoria migratorioides, Melanoplus spp., Schistocerca gregaria.
Aus der Ordnung der Blattaria z.B. Blatta orientalis, Periplaneta americana, Leucophaea maderae, Blattella germanica.
Aus der Ordnung der Dermaptera z.B. Forficula auricularia.
Aus der Ordnung der Isoptera z.B. Reticulitermes spp..
Aus der Ordnung der Phthiraptera z.B. Pediculus humanus corporis, Haematopinus spp., Linognathus spp., Trichodectes spp., Damalinia spp..
Aus der Ordnung der Thysanoptera z.B. Hercinothrips femoralis, Thrips tabaci, Thrips palmi, Frankliniella accidentalis.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuebniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp..
Aus der Ordnung der Siphonaptera z.B. Xenopsylla cheopis, Ceratophyllus spp..
Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp..

Die Wirkstoffkombinationen können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid

und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffkombinationen können in handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Auch eine Mischung mit anderen bekannten Wirkstoffen, wie Herbiziden oder mit Düngemitteln und Wachstumsregulatoren ist möglich.

Die erfindungsgemäßen Wirkstoffkombinationen können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnen sich die Wirkstoffkombinationen durch eine hervorragende Residualwirkung auf Holz und Ton sowie durch eine gute Alkalistabilität auf gekälkten Unterlagen aus.

Die erfindungsgemäßen Wirkstoffkombinationen wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.. Aus der Ordnung Diptera und den Unterordnungen Nematocerina sowie Brachycerina z. B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffkombinationen eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffkombinationen eine wirtschaftlichere und einfachere Tierhaltung möglich ist.

Die Anwendung der erfindungsgemäßen Wirkstoffkombinationen geschieht im Veterinärsektor in bekannter Weise durch enterale Verabreichung in Form von beispielsweise Tabletten, Kapseln, Tränken, Drenchen, Granulaten, Pasten, Boli, des feedthrough-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffkombinationen als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Wirkstoffkombinationen eine hohe insektizide Wirkung gegen Insekten zeigen, die technische Materialien zerstören.

Beispielhaft und vorzugsweise - ohne jedoch zu limitieren - seien die folgenden Insekten genannt:

Käfer wie

Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.

Hautflügler wie

Sirex juvencus, Urocerus gigas, Urocerus gigas taignus, Urocerus augur.

Termiten wie

Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:

Bauholz, Holzbalken, Eisenbahnschwellen, Brückenteile, Bootsstege, Holzfahrzeuge, Kisten, Paletten, Container, Telefonmasten, Holzverkleidungen, Holzfenster und - türen, Sperrholz, Spanplatten, Tischlerarbeiten oder Holzprodukte, die ganz allgemein beim Hausbau oder in der Bautischlerei Verwendung finden.

Die Wirkstoffkombinationen können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und

von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlomaphthalin, vorzugsweise $\alpha$-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Zugleich können die erfindungsgemäßen Wirkstoffkombinationen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflusskrebse) zusammengefasst werden, besondere Bedeutung zu.

Es wurde nun überraschenderweise gefunden, dass die erfindungsgemäßen Wirkstoffkombinationen eine hervorragende Antifouling (Antibewuchs)-Wirkung aufweisen.

Durch Einsatz der erfindungsgemäßen Wirkstoffkombinationen kann auf den Einsatz von Schwermetallen wie z.B. in Bis(trialkylzinn)-sulfiden, Tri-*n*-butylzinnlaurat, Tri*n*-butylzinnchlorid, Kupfer(I)-oxid, Triethylzinnchlorid, Tri-*n*-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-*n*-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfernaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:

Algizide wie
2-*tert.*-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;

Fungizide wie
Benzo[*b*]thiophencarbonsäurecyclohexylamid-S,S-dioxid, Dichlofluanid, Fluorfolpet, 3-Iod-2-propinyl-butylcarbamat, Tolylfluanid und Azole wie Azaconazole, Cyproconazole, Epoxyconazole, Hexaconazole, Metconazole, Propiconazole und Tebuconazole;

Molluskizide wie
Fentinacetat, Metaldehyd, Methiocarb, Niclosamid, Thiodicarb und Trimethacarb;

oder herkömmliche Antifouling-Wirkstoffe wie
4,5-Dichlor-2-octyl-4-isothiazolin-3-on, Düodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoffkombinationen in einer Konzentration von 0,001 bis 50 Gew.-%, insbesondere von 0,01 bis 20 Gew.-%.

Die erfindungsgemäßen Antifouling-Mittel enthalten desweiteren die üblichen Bestandteile wie z.B. in Ungerer, *Chem. Ind.* **1985**, *37*, 730-732 und Williams, Antifouling Marine Coatings, Noyes, Park Ridge, 1973 beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wässrigen System, Vinylchlorid/Vinylacetat-Copolymersysteme in Form wässriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten

Mischungen eingearbeitet werden.

Die Wirkstoffkombinationen eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge in Haushaltsinsektizid-Produkten verwendet werden. Sie sind gegen sensible und resistente Arten sowie gegen alle Entwicklungsstadien wirksam. Zu diesen

Schädlingen gehören:

Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dennaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Phthirus pubis.

Aus der Ordnung der Heteroptera z.B. Cimex hemipterus, Cimex lectularius, Rhodinus prolixus, Triatoma infestans.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gelund Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Erfindungsgemäß können alle Planzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließllch natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten, wie Spross, Blatt, Blüte und Wurzel verstanden werden, wobei beispielhaft, Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhiozome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.

B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die gute insektizide und akarizide Wirkung der erfindungsgemäßen Wirkstoffkombinationen geht aus den nachfolgenden Beispielen hervor. Während die einzelnen Wirkstoffe in der Wirkung Schwächen aufweisen, zeigen die Kombinationen eine Wirkung, die über eine einfache Wirkungssummierung hinausgeht.

Ein synergistischer Effekt liegt bei Insektiziden und Akariziden immer dann vor, wenn die Wirkung der Wirkstoffkombinationen größer ist als die Summe der Wirkungen der einzeln applizierten Wirkstoffe.

Die zu erwartende Wirkung für eine gegebene Kombination zweier Wirkstoffe kann nach S.R. Colby, Weeds 15 (1967), 20-22) wie folgt berechnet werden:

Wenn

X den Wirkungsgrad beim Einsatz des Wirkstoffes A in einer Aufwandmenge von m g/ha oder in einer Konzentration von m ppm bedeutet,

Y den Wirkungsgrad beim Einsatz des Wirkstoffes B in einer Aufwandmenge von n g/ha oder in einer Konzentration von n ppm bedeutet und

E den Wirkungsgrad beim Einsatz der Wirkstoffe A und B in Aufwandmengen von m und n g/ha oder in einer Konzentration von m und n ppm bedeutet,

dann ist

$$E = X + Y - \frac{X \cdot Y}{100}$$

Dabei wird der Wirkungsgrad in % ermittelt. Es bedeutet 0 % ein Wirkungsgrad, der demjenigen der Kontrolle entspricht, während ein Wirkungsgrad von 100 % bedeutet, dass kein Befall beobachtet wird.

Ist die tatsächliche Wirkung größer als berechnet, so ist die Kombination in ihrer Wirkung überadditiv, d.h. es liegt ein synergistischer Effekt vor. In diesem Fall muss der tatsächlich beobachtete Wirkungsgrad größer sein als der aus der oben angeführten Formel errechnete Wert für den erwarteten Wirkungsgrad (E).

**Anwendungsbeispiele**

**Beispiel A**

Heliothis virescens-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Sojatriebe (Glycine max) werden durch Tauchen in die Wirkstoffzubereitung der gewünschten Konzentration behandelt und mit Heliothis virescens-Raupen besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden. Die ermittelten Abtötungswerte verrechnet man nach der Colby-Formel.

Bei diesem Test zeigt z. B. die folgende Wirkstoffkombination gemäß vorliegender Anmeldung eine synergistisch verstärkte Wirksamkeit im Vergleich zu den einzeln angewendeten Wirkstoffen:

Tabelle A

| pflanzenschädigende Insekten<br>Heliothis virescens-Test | | |
|---|---|---|
| **Wirkstoffe** | **Wirkstoffkonzentration in ppm** | **Abtötungsgrad in % nach $3^d$** |
| Bsp. (I-b-1) bekannt | 0,32 | 0 |
| Betacyfluthrin bekannt | 0,32 | 75 |
| Bsp. (I-b-1) + Betacyfluthrin (1:1) erfindungsgemäß | 0,32 + 0,32 | gef.* 100 ber.** 75 |
| * gef. = gefundene Wirkung<br>** ber. = nach der Colby-Formel berechnete Wirkung | | |

**Beispiel B**

Nephotettix-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Reiskeimlinge (Oryza sativa) werden durch Tauchen in die Wirkstoffzubereitung der gewünschten Konzentration behandelt und mit der Grünen Reiszikade (Nephotettix cincticeps) besetzt, solange die Blätter noch feucht sind.

Nach der gewünschten Zeit wird die Abtötung in % bestimmt. Dabei bedeutet 100 %, dass alle Zikaden abgetötet wurden; 0 % bedeutet, dass keine Zikaden abgetötet wurden. Die ermittelten Abtötungswerte verrechnet man nach der Colby-Formel.

Bei diesem Test zeigt z. B. die folgende Wirkstoffkombination gemäß vorliegender Anmeldung eine synergistisch verstärkte Wirksamkeit im Vergleich zu den einzeln angewendeten Wirkstoffen:

Tabelle B

| Pflanzenschädigende Insekten<br>Nephotettix-Test | | |
|---|---|---|
| **Wirkstoffe** | **Wirkstoffkonzentration in ppm** | **Abtötungsgrad in % nach $6^d$** |
| Bsp. (I-b-1) bekannt | 0,32 | 0 |
| Betacyfluthrin bekannt | 0,32 | 50 |
| Bsp. (I-b-1) + Betacyfluthrin (1:1) erfindungsgemäß | 0,32 + 0,32 | gef.* 85 ber.** 50 |
| * gef. = gefundene Wirkung<br>** ber. = nach der Colby-Formel berechnete Wirkung | | |

**Beispiel C**

**Plutella-Test**

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.
Kohlblätter (Brassica oleracea) werden durch Tauchen in die Wirkstoffzubereitung der gewünschten Konzentration behandelt und mit Raupen der Kohlschabe (Plutella xylostella) besetzt, solange die Blätter noch feucht sind.
Nach der gewünschten Zeit wird die Abtötung in % bestimmt. Dabei bedeutet 100 %, dass alle Raupen abgetötet wurden; 0 % bedeutet, dass keine Raupen abgetötet wurden. Die ermittelten Abtötungswerte verrechnet man nach der Colby-Formel.
Bei diesem Test zeigt z. B. die folgende Wirkstoffkombination gemäß vorliegender Anmeldung eine synergistisch verstärkte Wirksamkeit im Vergleich zu den einzeln angewendeten Wirkstoffen:

Tabelle C

| **pflanzenschädigende Insekten**<br>Plutella-Test | | |
|---|---|---|
| **Wirkstoffe** | **Wirkstoffkonzentration in ppm** | **Abtötungsgrad in % nach 3[d]** |
| Bsp. (I-b-1) bekannt | 1,6 | 0 |
| Betacyfluthrin bekannt | 1,6 | 80 |
| Bsp. (I-b-1) + Betacyfluthrin (1:1) erfindungsgemäß | 1,6 + 1,6 | gef.* 100 / ber.** 80 |
| * gef. = gefundene Wirkung<br>** ber. = nach der Colby-Formel berechnete Wirkung | | |

**Beispiel D**

**Spodoptera exigua-Test**

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.
Kohlblätter (Brassica oleracea) werden durch Tauchen in die Wirkstoffzubereitung der gewünschten Konzentration behandelt und mit Raupen des Heerwurms (Spodoptera exigua) besetzt, solange die Blätter noch feucht sind.
Nach der gewünschten Zeit wird die Abtötung in % bestimmt. Dabei bedeutet 100 %, dass alle Raupen abgetötet wurden; 0 % bedeutet, dass keine Raupen abgetötet wurden. Die ermittelten Abtötungswerte verrechnet man nach der Colby-Formel.
Bei diesem Test zeigt z. B. die folgende Wirkstoffkombination gemäß vorliegender Anmeldung eine synergistisch verstärkte Wirksamkeit im Vergleich zu den einzeln angewendeten Wirkstoffen:

Tabelle D

| **pflanzenschädigende Insekten**<br>Spodoptera exigua-Test | | |
|---|---|---|
| **Wirkstoffe** | **Wirkstoffkonzentration in ppm** | **Abtötungsgrad in % nach 6[d]** |
| Bsp. (I-b-1) bekannt | 8 | 0 |
| Betacyfluthrin bekannt | 8 | 90 |
| Bsp. (I-b-1) + Betacyfluthrin erfindungsgemäß | 8 + 8 | gef.* 100 / ber.** 90 |
| * gef. = gefundene Wirkung<br>** ber. = nach der Colby-Formel berechnete Wirkung | | |

**Beispiel E**

**Tetranychus-Test**

(OP-resistent/Spritzbehandlung)

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.
Bohnenpflanzen (Phaseolus vulgaris), die stark von allen Stadien der gemeinen Spinnmilbe (Tetranychus urticae) befallen sind, werden mit einer Wirkstoffzubereitung der gewünschten Konzentration gespritzt.
Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden. Die ermittelten Abtötungswerte verrechnet man nach der Colby-Formel.
Bei diesem Test zeigen z.B. die folgenden Wirkstoffkombinationen gemäß vorliegender Anmeldung einesynergistisch verstärkte Wirksamkeit im Vergleich zu den einzeln angewendeten Wirkstoffen:

Tabelle E

| **Pflanzenschädigende Milben**<br>Tetranychus-Test (OP-resistent/Spritzbehandlung) | | |
|---|---|---|
| **Wirkstoffe** | **Wirkstoffkonzentration in ppm** | **Abtötungsgrad in % nach 14$^{d}$** |
| Bsp. (I-b-1) bekannt | 0,32 | 0 |
| Methamidophos bekannt | 0,32 | 0 |
| Bsp. (I-b-1) + Methamidophos (1:1) erfindungsgemäß | 0,32 + 0,32 | gef.* 90 ber.** 0 |
| * gef. = gefundene Wirkung<br>** ber. = nach der Colby-Formel berechnete Wirkung | | |

**Patentansprüche**

1. einfügen von

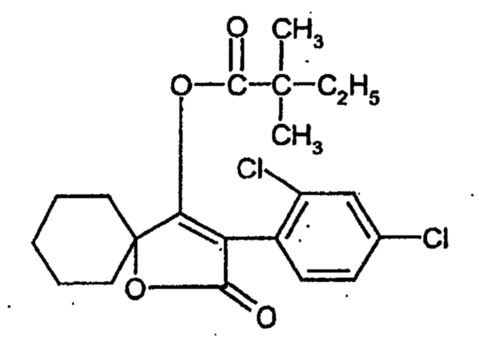

(I-b-1).

und mindestens eine der nachfolgenden Verbindungen

2-(Acetyloxy)-3-dodecyl-1,4-naphthalinidon
Acephat

Acrinathrin
Alpha-Cypermethrin
Amitraz
Azadirachtin
Azinphosmethyl
Betacyfluthrin
Bromopropylat
Buprofezin
Carbaryl
Chinomethionat
Chlorfenvinphos
Chlorfluazuron
Chlorpyrifos
Cyhalothrin
Cypermethrin
Cyromazin
Deltamethrin
Diazinon
Dichlorphos
Dicofol
Dicrotophos
Diflubenzuron
Dimethoat
Diofenolan
Disulfoton
Emamectin
Endosulfan
Esfenvalerat
Ethion
Etofenprox
Fenazaquin
Fenitrothion
Fenoxycarb
Fenpropathrin
Fenpyrad (Tebufenpyrad)
Fenthion
Fenvalerat
Fipronil
Flucythrinat
Formetanat
Hexythiazox
Indoxacarb
Isoxathion
Ivermectin
Lambda-Cyhalothrin
Lindan (Gamma-HCH)
Lufenuron
Malathion
Methamidophos
Methidathion
Methiocarb
Methomyl
Methoxyfenozid
Mevinphos
Milbemectin
Monocrotophos
Oxamyl
Oxydemeton-methyl

Parathion
Parathion-methyl
Permethrin
Phenthoat
Phorat
Phosalon
Phosmet
Phosphamidon
Phoxim
Pirimicarb
Pirimiphos-methyl
Profenophos
Propargit
Propoxur
Prothiophos
Pymetrozin
Pyrimidifen
Pyriproxyfen
Tau-fluvalinat
Tebufenozid
Tebupyrimphos
Teflubenzuron
Tetradifon
Thiocyclam
Thiodicarb
Tralomethrin
Triarathen
Triazamat
Triazophos
Trichlorfon
Trichogramma spp.
Triflumuron
Verticillium- lecanii
Zeta-Cypermethrin

**2.** Verwendung von Mischungen, wie in Anspruch 1, definiert, zur Bekämpfung tierischer Schädlinge.

**3.** Verfahren zur Bekämpfung tierischer Schädlinge, **dadurch gekennzeichnet, dass** man Mischungen, wie in Anspruch 1, definiert, auf tierische Schädlinge und/oder deren Lebensraum einwirken lässt.

**4.** Verfahren zur Herstellung insektizider und akarizider Mittel, die **dadurch gekennzeichnet sind, dass** man Mischungen, wie in Anspruch 1, definiert, mit Streckmitteln und/oder oberflächenaktiven Stoffen vermischt.

## Claims

**1.** Composition comprising the compound of the formula (I-b-1)

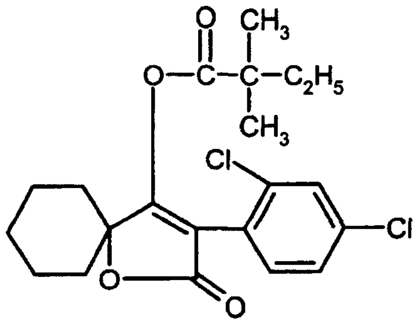

(I-b-1)

and at least one of the following compounds

2-(acetyloxy)-3-dodecyl-1,4-naphthalenedione
Acephate
acrinathrin
alpha-cypermethrin
Amitraz
azadirachtin
azinphos-methyl
betacyfluthrin
bromopropylate
buprofezin
carbaryl
quinomethionate
chlorfenvinphos
chlorfluazuron
chlorpyrifos
cyhalothrin
cypermethrin
cyromazin
deltamethrin
diazinon
dichlorphos
dicofol
dicrotophos
diflubenzuron
dimethoate
diofenolan
disulfoton
emamectin
endosulfan
esfenvalerate
ethion
etofenprox
fenazaquin
fenitrothion
fenoxycarb
fenpropathrin
fenpyrad (tebufenpyrad)
fenthion
fenvalerate
fipronil
flucythrinate
formetanate
hexythiazox

indoxacarb
isoxathion
ivermectin
lambda-cyhalothrin
lindane (gamma-HCH)
lufenuron
malathion
methamidophos
methidathion
methiocarb
methomyl
methoxyfenozide
mevinphos
milbemectin
monocrotophos
oxamyl
oxydemeton-methyl
parathion
parathion-methyl
permethrin
phenthoate
phorate
phosalone
phosmet
phosphamidon
phoxim
pirimicarb
pirimiphos-methyl
profenophos
propargite
propoxur
prothiophos
pymetrozin
pyrimidifen
pyriproxyfen
tau-fluvalinate
tebufenozide
tebupirimphos
teflubenzuron
tetradifon
thiocyclam
thiodicarb
tralomethrin
triarathene
triazamate
triazophos
trichlorfon
trichogramma spp.
triflumuron
Verticillium lecanii
zeta-cypermethrin.

**2.** Use of mixtures as defined in Claim 1 for controlling animal pests.

**3.** Method of controlling animal pests, **characterized in that** mixtures as defined in Claim 1 are allowed to act on animal pests and/or their environment.

**4.** Process for the preparation of insecticidal and acaricidal compositions, **characterized in that** mixtures as defined

in Claim 1 are mixed with extenders and/or surfactants.

## Revendications

**1.** Agent contenant le composé de la formule (I-b-1) :

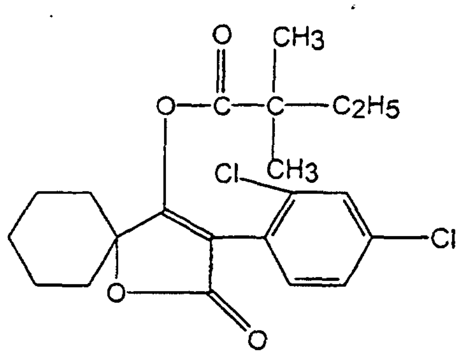

(I-b-1)

et au moins un des composés suivants :

la 2-(acétyloxy)-3-dodécyl-1,4-naphtalinidone,
l'acéphate,
l'acrinathrine,
l'α-cyperméthrine,
l'amitraz,
l'azadirachtine,
l'azinphos-méthyle,
la bêtacyfluthrine,
le bromopropylate,
la buprofézine,
le carbaryle,
le quinométhionate,
le chlorofenvinphos,
le chlorofluazuron,
le chlorpyrifos,
la cyhalothrine,
la cyperméthrine,
la cyromazine,
le deltaméthrine,
la diazinone,
le dichlorphos,
le dicofol,
le dicrotophos,
le diflubenzuron,
le diméthoate,
le diofenolan,
le disulfoton,
l'énamectine,
l'endosulfane,
l'esfenvalérate,
l'éthion,
l'éthofenprox,
le fenazaquin,
le fentrothion,
le fénoxycarbe,

la fenpropathrine,
le fenpyrade (tébufenpyrade),
le fenthion,
le fenvalérate,
le fipronile,
le flucythrinate,
le formétanate,
l'hexythiazox,
l'indoxacarbe,
l'isoxathion,
l'ivermectine,
la lambda-cyhalothrine,
le lindane (gamma-HCH),
le lufénuron,
la malathion,
le métamidophos,
le méthidathion,
le méthiocarbe,
le méthomyle,
le méthoxyfénozide,
le mevinphos,
la milbémectine,
le monocrotophos,
l'oxamyle,
l'oxydéméton-méthyle,
le parathion,
le parathion-méthyle,
la perméthrine,
le phentoate,
le phorate,
le phosalon,
le phosmet,
le phosphamidon,
le phoxim,
le pirimicarbe,
le pirimiphos-méthyle,
le profenophos,
le propargite,
le propoxur,
le prothiophos,
la pymétrozine,
le pyrimidifène,
le pyriproxyfène,
le tau-fluvalinate,
le tébufénbozide,
le tébupyrimphos,
le téflubenzuron,
le tétradifon,
le thiocyclam,
le thiodicarbe,
la tralométhrine,
le triarathène,
le triazamate,
le triazophos,
le trichlorfon,
*Trichogramma* spp.,
le triflumuron,
*Verticillium lecanii*,

la dzêta-cyperméthrine.

2. Utilisation de mélanges tels que définis à la revendication 1, pour lutter contre les parasites animaux.

3. Procédé pour lutter contre les parasites animaux, **caractérisé en ce que** l'on fait agir des mélanges tels que définis à la revendication 1, sur les parasites animaux et/ou leur biotope.

4. Procédé de préparation d'un agent insecticide et acaricide, qui est **caractérisé en ce que** l'on malaxe des mélanges tels que définis à la revendication 1, avec des diluants et/ou tensioactifs.